# FASCICULE DE BREVET EUROPEEN

(11) **EP 1 742 676 B1**
(45) Date de publication et mention de la délivrance du brevet: **13.04.2011**
(21) Numéro de dépôt: 05763676.3
(22) Date de dépôt: 29.04.2005
(51) Int. Cl.: A61L 24/00, A61L 27/42

(54) **COMPOSITION POUR CIMENT INJECTABLE, UTILE COMME SUBSTITUT OSSEUX**
ZUSAMMMENSETZUNG FÜR INJIZIERBAREN ZEMENT ALS KNOCHENERSATZ
COMPOSITION FOR INJECTABLE CEMENT USEFUL AS BONE REPLACEMENT

(30) Priorité: 03.05.2004 FR 0404714
(43) Date de publication de la demande: 17.01.2007
(73) Titulaire: CENTRE NATIONAL DE LA RECHERCHE SCIENTIFIQUE, 75016 Paris Cédex (FR); Université de Montpellier I, 34006 Montpellier Cedex 1 (FR)
(72) Inventeur: BOUDEVILLE, Philippe, F-34830 CLAPIERS (FR); VERT, Michel, F-34170 CASTELNAU-LE-LEZ (FR); MUNIER, Sylvie, F-34090 MONTPELLIER (FR)
(74) Mandataire: Sueur, Yvette
(86) Numéro de dépôt international: PCT/FR2005/001078
(87) Numéro de publication internationale: WO 2005/115488

(56) Documents cités:
- WO-A1-03/103734
- US-B1- 6 593 394

## Description

La présente invention concerne une composition pour ciment injectable utile comme substitut osseux.

Les ciments hydrauliques phosphocalciques sont utiles comme substituts osseux. Les ciments injectables à l'aide d'une seringue sont particulièrement recherchés, dans la mesure où ils permettent d'effectuer le comblement d'un défaut osseux, par une chirurgie peu invasive. En outre, le renforcement et la stabilisation d'un tissu osseux ostéoporotique peut être effectué uniquement par injection, sans exérèse ou destruction de la trame osseuse résiduelle.

Il est connu de préparer un ciment hydraulique phosphocalcique en mélangeant de l'eau ou une phase aqueuse avec une poudre constituée par divers précurseurs de phosphate calcique. Par exemple, M. Bohner (Injury, 2000, 31:S-D37-47) décrit la préparation de ciments hydrauliques à partir d'un mélange d'un phosphate calcique à caractère acide et d'un phosphate de calcium à caractère basique. De tels ciments sont commercialisés notamment sous les dénominations BoneSource®, Cementek®, Calcibon® ou ChronoS Inject®. L'un des inconvénients de ces ciments est qu'ils se résorbent très lentement.

Diverses voies ont été explorées pour améliorer la vitesse de résorption des ciments hydrauliques.

Il a été envisagé de remplacer partiellement le calcium des composés phosphocalciques par du strontium. Ainsi GB-943678 décrit des ciments hydrauliques phosphocalciques obtenus à partir d'une poudre constituée par un mélange de M(H₂PO₄)₂ anhydre et par un oxyde d'un métal M' (M et M' pouvant représenter indépendamment l'un de l'autre Zn, Mg, Ba, Ca, Al, Cd ou Sr) en proportion massique à peu près égale, ladite poudre étant mélangée à de l'eau dans un rapport eau/poudre de 0,15 ml/g. Les seules compositions illustrées sont Ca(H₂PO₄)₂ + ZnO d'une part, Zn(H₂PO₄)₂ + ZnO d'autre part. Les ciments obtenus à partir de ces poudres ont une résistance à la compression supérieure à 100 MPa et des temps de prise de l'ordre de 8-10 min. Cependant, les inventeurs de la présente invention ont effectué des essais avec un mélange de bis-dihydrogénophosphate de calcium anhydre (Ca(H₂PO₄)₂, et de CaO avec un rapport L/P de 1 ml/g, afin d'obtenir une pâte qui peut être travaillée dans les conditions décrites dans GB-943678 et les résultats obtenus montrent que la résistance à la compression après 24 h est inférieure à 1 MPa. L'enseignement général de ce document ne permet donc pas d'obtenir un ciment présentant les propriétés requises (bonne résistance à la compression, grande vitesse de prise) pour n'importe quelle combinaison M, M'. En outre, dans le cas particulier dans lequel l'un des M ou M' serait Sr (non illustré dans GB943678), ni le composé Sr(H₂PO₄)₂, ni le composé SrO ne permettrait la formation d'une porosité moyenne.

Leroux, et al, (Bioceramics, 13, Trans Tech Publications, Switzerland, 2001, 235-238) décrit un ciment contenant jusqu'à 4,3% en poids de Sr, correspondant à une composition finale de ciment Ca_{9,75},Sr_{0,25}(PO₄)₆(OH)₂. Cette composition peut être obtenue en ajoutant du nitrate de strontium Sr(NO₃)₂ à la phase liquide du ciment Cementek® précité, dont la phase solide est constituée par un mélange de α-Ca₃(PO₄)₂ (ou α-TCP), de phosphate de tétracalcium (ou TTCP) et de glycérophosphate. Ici également, le choix particulier du précurseur de strontium ne permet pas d'obtenir une porosité moyenne.

Une autre solution envisagée pour améliorer la vitesse de résorption du substitut osseux repose sur le principe que la présence de macropores interconnectés dans le matériau phosphocalcique favorise la résorption passive par dissolution de l'implant en permettant sa percolation par les fluides biologiques, et la résorption active par les ostéoclastes en permettant leur pénétration dans les macropores qui ont une dimension suffisante, c'est-à-dire supérieure à 100 µm. US-6,547,866 décrit des matériaux phosphocalciques poreux obtenus en ajoutant à la composition des composés libérant un gaz lors de la prise du ciment (carbonate + acide citrique qui libèrent CO₂, peroxyde d'hydrogène qui libère O₂). Cependant, les pores obtenus ont une dimension moyenne relativement faible et la résistance mécanique est diminuée. Il a en outre été proposé composition avant sa prise, d'un composé hydrosoluble du type NaHCO₃, Na₂HPO₄ saccharose ou mannitol (Markovic, et al., Bioceramics, Trans Tech Publications, Switzerland, 2001, 773-775 ; Takagi and Chow, J. Mater. Sci. Mater. Mad. 2001 ;13 :135-139). La dissolution de ces sucres gène la prise du ciment, ce qui diminue fortement la résistance à l'écrasement.

On a également envisagé l'addition de particules ou de fibres d'un polymère biodégradable. Simon, et al, (J. Orthop. Res. 2002 ; 25 : 473-482) décrivent des ciments du type BoneSource^{®}, obtenus à partir de TTCP qui est un phosphate basique et de phosphate de dicalcium anhydrique (DCPA) qui est un phosphate acide, et des microsphères de poly-lactide-co-glycolide PLAGA, la décomposition rapide dudit polymère donnant une macroporosité. Cependant, la partie minérale n'est pas dégradée in vitro après 90 jours.

Xu H, et al, (Biomaterials 2002 ; 23 :193-202 et Biomaterials 2004 ; 25 :1029-1037) décrivent un ciment de phosphate de calcium (BoneSource®) contenant des fibres de Vicryl® ou un filet de Vicryl® qui est un polymère biorésorbable très riche en acide glycolique. Du fait de la présence des fibres ou du filet, ce ciment n'est pas injectable.

Ruhe, et al, (J. Bone Joint Surg. 2003 ; 85 : 75-81) décrivent un ciment Calcibon® (α-TCP+DCPA+CaCO₃+HA) contenant des microsphères de PLAGA chargées en rhBMP-2 qui diffuse à partir du polymère (HA désignant l'hydroxyapatite). Cependant, les auteurs indiquent que l'introduction du polymère diminue très fortement la résistance à l'écrasement qui passe de 38,6 à 6,4 MPa (valeur inférieure à celle de l'os trabéculaire ≈ 10 MPa). Mais les auteurs n'indiquent pas si, après 28 jours en solution tampon pH 7, le polymère et/ou la partie minérale du ciment sont dégradés.

WO-03/103734 décrit une composition de ciment utile notamment pour le comblement osseux. Ladite composition contient un mélange de poudres contenant un précurseur de calcium [en particulier Ca₃(PO₄)₂], un précurseur de strontium (en particulier SrHPO₄ et/ou Sr₃(PO₄)₂ et éventuellement SrCO₃], et un orthophosphate, dans une solution aqueuse d'un sel de métal alcalin ou d'ammonium de l'acide orthophosphorique.

US-6,593,394 décrit une composition de ciment utilisable comme substituant osseux. Ladite composition comprend une poudre contenant du phosphate de strontium et du phosphate de calcium, et un composant liquide contenant une résine de diméthacrylate de bisphénol A et de diglycidyléther, les quantités de phosphates étant telles que le rapport molaire Sr/(Sr+Ca) soit d'au moins 10%.

La présente invention a pour but de fournir un ciment injectable utilisable comme substitut osseux, qui présente un temps d'injection et un temps de prise compatibles avec les techniques d'injection, une bonne résistance à la compression après injection et une vitesse de résorption élevée après injection.

C'est pourquoi la présente invention a pour objet une composition pour ciment injectable biocompatible et biodégradable, un procédé de préparation d'un ciment injectable à partir de ladite composition, le ciment injectable obtenu, ainsi que son utilisation par injection dans une matière osseuse, notamment pour le traitement de l'ostéoporose, pour la vertébrothérapie, pour le comblement osseux après une fracture ou une exérèse.

La composition pour ciment injectable selon l'invention comprend une phase solide minérale et une phase liquide séparées, et éventuellement des microparticules d'un polymère biocompatible et biodégradable,
- la phase solide minérale étant constituée par un mélange de poudres ayant la composition molaire (CP)₆ (CaO)_{y} (SrCO₃)_{z}, dans laquelle CP représente l'hydrogénophosphate de calcium dihydraté CaHPO₄, 2H₂O (DCPD), l'hydrogénophosphate de calcium anhydre CaHPO₄ (DCPA), l'hydrogénophosphate mixte de calcium et de strontium anhydre (Ca₁₋ₓSrₓ) HPO₄, un mélange équimolaire de bis-dihydrogénophosphate de calcium monohydraté Ca(H₂PO₄)₂, H₂O (MCPM) et d'oxyde de calcium CaO, ou un mélange de deux ou trois de ces composés, et y+z = 4±1 ;
- la phase liquide étant constituée par de l'eau pure apyrogène ou par une solution aqueuse saline ayant un pH de 4 à 9;
le rapport L/P [volume de phase liquide /masse de phase solide minérale] étant au moins égal à 0,4 ml/g.

Le rapport L/P est de préférence de 0,4 ml/g à 0,7 ml/g en l'absence de microparticules de polymère (plus particulièrement de 0,5 à 0,6 ml/g). Le rapport L/P est au moins égal à 0,6 ml/g en présence de microparticules de polymère.

Le mélange de MCPM et de CaO est un précurseur de DCPD. Les deux constituants du mélange réagissent avec précipitation de DCPD dans les 3 minutes suivant la mise en contact de la phase solide avec la phase liquide.

Les compositions dans lesquelles au moins une partie du constituant CP représente DCPD sont préférées. On préfère tout particulièrement les compositions dans lesquelles CP est constitué uniquement par DCPD.

Lorsque la phase liquide est une solution saline, elle peut être constituée par :
- une solution contenant de 0,4 à 1 mol/l d'un mélange de NaH₂PO₄ et de Na₂HPO₄ 12H₂O (tampon NaP), par exemple une solution de pH 7 contenant 0,75 mol/l de phosphate ;
- une solution aqueuse ayant un pH de 4 à 8 et contenant de 0,4 à 1 mol/l d'un mélange de NaH₂PO₄ et de glycérophosphate de sodium hexahydraté Na₂PO₄C₃H₇O₂, 6H₂O (tampon NaGP), par exemple une solution de pH 6,6 contenant 0,75 mol/l de phosphate ;
- une solution aqueuse contenant de 0,4 à 1,5 mol/l d'un mélange de NH₄H₂PO₄ et de (NH₄)₂HPO₄ (tampon NH4P), par exemple une solution de pH 6,65 contenant 0,75 mol/l de phosphate.

Dans un mode de réalisation particulier, la composition pour ciment injectable contient en outre des microparticules d'un polymère biocompatible et biodégradable. A titre d'exemple, on peut citer un polymère PLAGA intrinsèquement amorphe, constitué par des unités L-lactique, des unités D-lactique, et des unités glycoliques. On peut également citer un polymère PLA constitué uniquement par des unités L-lactiques. D'autres polymères, dégradables par hydrolyse ou biodégradables par action de cellules vivantes, peuvent être utilisés, par exemple des copolymères d'acide lactique, d'acide glycolique et d'acide gluconique qui sont décrits notamment dans FR-2765228 et qui se dégradent plus rapidement que le PLAGA à conditions identiques. Le rapport massique du constituant polymère à la phase solide minérale est compris entre 20 et 60 %, de préférence de l'ordre de 40%.

Dans une mode de réalisation préféré d'une composition pour ciment injectable qui contient des microparticules de polymère, la composition comprend une troisième phase constituées par lesdites microparticules. Dans un autre mode de réalisation, la phase minérale solide contient en outre lesdites microparticules.

La présence de microparticules de polymère dans une composition pour ciment injectable n'entraîne aucune diminution de la résistance à l'écrasement du ciment après prise, la résistance restant supérieure à celle de l'os trabéculaire.

Les microparticules sont de préférence des microsphères. Elles peuvent contenir éventuellement un composé chimique ou biologique, qui sera libéré lors de la dégradation du polymère après l'implantation in vivo du ciment injectable. Le diamètre des microsphères peut être compris entre 50 et 500 µm, de préférence entre 125 et 250 µm.

Lorsque la composition pour ciment injectable contient des microparticules de polymère, il est préférable d'augmenter la valeur du rapport L/P, afin de maintenir le temps d'injection total à une valeur suffisante. Le rapport L/P est dans ce cas au moins égal à 0,6 ml/g. L'augmentation de la proportion de phase liquide a pour but de compenser l'absorption d'une certaine quantité de liquide par le polymère, et de maintenir suffisamment de phase liquide dans la phase solide minérale pour qu'elle reste injectable.

Le procédé de préparation du ciment injectable à partir de la composition selon l'invention consiste à mettre en contact la phase solide (qui contient éventuellement des microparticules de polymère) et la phase liquide de la composition pour ciment injectable, au moment de l'utilisation du ciment injectable, dans les conditions d'asepsie requises pour l'injection de substituts osseux. Le procédé peut être mis en oeuvre à l'aide de tout dispositif classique pour la préparation d'un ciment. On peut citer par exemple le dispositif de mélange décrit dans US-5,549,380 qui permet d'introduire la phase liquide dans la phase solide par aspiration au moment où le mélange doit être effectué. On peut également utiliser une poche souple à deux compartiments contenant chacun l'une des phases de la composition, les compartiments étant séparés par un opercule qui est brisé au moment où le mélange doit être effectué, permettant le passage de la phase liquide dans le compartiment contenant la phase solide.

Lorsque la composition pour ciment injectable comprend des microparticules de polymère sous forme d'une 3^{ème} phase, cette 3^{ème} phase est ajoutée à la pâte obtenue immédiatement après la mise en contact de la phase liquide et de la phase minérale solide.

En l'absence de microparticules de polymère, la pâte obtenue immédiatement après mise en contact des deux phases de la composition selon l'invention est constituée par (CP)_{6,} (CaO)_{y} et (SrCO₃)_{z}, de l'eau et les sels éventuellement contenus dans la phase liquide, les quantités respectives étant telles que :
- Le rapport molaire (CP) / (CaO) / (SrCO₃) est de 6/y/z avec y+z = 4±1,
- Le rapport L/P (volume de phase liquide / masse de phase solide minérale) est de 0,4 à 0,7 ml/g, plus particulièrement de 0,5 à 0,6 ml/g.

Le mode de réalisation dans lequel CP contient CaHPO₄, 2H₂O est préféré, en particulier dans lequel CP est constitué uniquement par CaHPO₄, 2H₂O.

La pâte injectable ainsi obtenue a un temps d'injection et un temps de prise compatibles avec une utilisation comme substitut osseux par injection. Par temps d'injection, on entend le temps pendant lequel une composition reste totalement injectable, après mélange de la phase liquide et la phase solide. Par temps de prise, on entend la durée nécessaire pour que la composition, après mélange des deux phases, devienne solide (Norme ASTM C191).

Le temps de prise du ciment est plus court si l'on utilise une solution tampon NaP ou NH4P. Le temps d'injection est plus long avec une solution tampon NaGP.

Le ciment injectable selon l'invention donne après injection, un matériau constitué majoritairement par de l'hydroxyapatite modifiée Ca₁₀-xSrₓ(PO₄)₆(OH)₂, qui contient en outre une faible quantité de phosphate octacalcique et éventuellement des traces de SrCO₃ n'ayant pas réagi. Il présente une porosité caractérisée par des pores ayant une dimension moyenne de l'ordre de 20 à 50 µm provenant essentiellement de la décomposition de SrCO₃ lors de la prise du ciment. Cette porosité moyenne permet une meilleure pénétration des fluides à l'intérieur du ciment, une augmentation de la surface spécifique d'échange qui facilite la diffusion des ions provenant de la dissolution des composants du ciment, et qui provoque donc une meilleure résorption passive.

Lorsque la composition pour ciment injectable contient en outre des microparticules de polymère biodégradable biorésorbable, le matériau obtenu après injection contient les mêmes constituants que ci-dessus, ainsi que des microparticules de polymères, le rapport massique polymère/phase minérale solide étant entre 20 et 60% (de préférence de l'ordre de 40%) et le rapport L/P initial étant au moins égal à 0,6 ml/g. On constate en outre que la porosité moyenne mentionnée précédemment existe à un degré supérieur autour des microparticules de polymère qui génèrent des composés acides lors de la dégradation hydrolytique, ce qui constitue un avantage pour la dégradation in vivo. Après le temps nécessaire à la résorption du polymère, il présente en outre un réseau de macropores interconnectés ayant une dimension moyenne supérieure à 100 µm.

Lorsque le polymère est le PLAGA précité, les microparticules sphériques de PLAGA peuvent être obtenues en ajoutant une solution de PLAGA dans le dichlorométhane à une solution aqueuse d'alcool polyvinylique (PVA), et en maintenant le mélange sous agitation pendant une certaine durée, puis en récupérant les particules par filtration. Des particules non sphériques de PLAGA peuvent être obtenues par cryobroyage.

En solution, tamponnée ou non, un ciment selon l'invention libère de façon continue des ions strontium et peut donc agir comme système à libération contrôlée et prolongée d'ions strontium qui activent la reconstruction osseuse par stimulation des ostéoblastes.

Lorsque le ciment contient des microsphères d'un polymère, ledit polymère se dégrade d'abord lentement, puis beaucoup plus rapidement à partir de 3 semaines. Cette dégradation du polymère pourra être mise à profit, en chargeant celui-ci d'un principe actif (par exemple un antibiotique) ou d'une protéine (par exemple un facteur de croissance), pour obtenir également une libération contrôlée de ce principe actif dont l'action pourra être complémentaire de celle du strontium. On peut notamment combiner avantageusement les particules de PLAGA ou d'autres polymères dégradables avec des agents antitumoraux, notamment dans le cas de comblement d'exérèse osseuse post tumorale.

La présence des ions Sr²⁺ rend le ciment plus radioopaque, ce qui facilite le suivi par radioscopie lors de l'injection du ciment dans la matière osseuse.

Un ciment injectable selon l'invention qui ne contient pas de particules de polymère est utilisable avantageusement pour la stabilisation et/ou le renforcement de l'os ostéoporotique par injection dans la trame osseuse résiduelle, par exemple pour la vertébroplastie, la présence de Sr²⁺ favorisant l'ostéogénèse.

Un ciment injectable contenant des microparticules de polymère peut être utilisé pour le comblement de défauts osseux résultant d'une exérèse ou d'une fracture. La libération de Sr²⁺ et la macroporosité créée par la dégradation du polymère in vivo stimule la repousse osseuse. En outre, du fait que le strontium possède un isotope radioactif qui est utilisé dans le traitement de certaines formes de cancer à tropisme osseux, le carbonate de strontium inactif peut être remplacé totalement ou partiellement par du carbonate de strontium 89 radioactif, pour le comblement après exérèse d'une tumeur osseuse avec éventuellement une substance antimitotique chargée dans le polymère biodégradable.

La présente invention est illustrée par les exemples suivants, auxquels elle n'est cependant pas limitée.

### Exemple 1

### Ciment injectable sans microbilles de polymère

On a préparé un mélange de poudre ayant la composition molaire suivante : 6 DCPD, 2,5 CaO, 1,5 SrCO₃.

On a préparé :
- une solution aqueuse du type tampon NaP ayant la composition NaH₂PO₄ + Na₂HPO₄, 12H₂O (0,75 M), et un pH de 7 ;
- une solution aqueuse du type tampon NaGP ayant la composition NaH₂PO₄ + Na₂PO₄C₃H₇O₂, 6H₂O (0, 75 M), et un pH de 6,6
- une solution aqueuse du type tampon NH4P ayant la composition NH₄H₂PO₄ + (NH₄)₂HPO₄ (0,75 M), et un pH de 6,65.

Avec chacune des solutions tampon, on a préparé deux compositions pour ciment, en mélangeant la solution et la poudre dans un rapport phase liquide/phase solide minérale L/P (en ml/g) de 0,5 et de 0,6.

Les caractéristiques des compositions obtenues sont données dans le tableau I ci-après.

**Tableau 1**

| **Echantillon** | **1A** | **1B** | **1C** | **1D** | **1E** |
|---|---|---|---|---|---|
| Phase liquide | NaP | NaP | NaGP | NaGP | NH4P |
| L/P | 0,5 | 0,6 | 0,5 | 0,6 | 0,5 |
| Résistance à la compression (Mpa) | 21,0±2,8 | 17,6±1,8 | 19,5±2,3 | 17,6±3.2 | 18,0±0,5 |
| Résist. à la compr. diamétrale (Mpa) | 1,6±0,7 | 1,2±0,16 | 2,3±0,4 | 1,5±0,1 | 1,5±0,2 |
| Temps de prise initial (min) | | 16 | | | |
| Temps de prise final (min) | 14 | 19 | 19 | 30 | 15±1 |
| Temps d'injection t_{100%} (min) | 6±0,5 | 12±1 | 13±1 | 21±3 | 9±1 |
| Température maximale atteinte (°C) | 41,5 | | 39,2 | | 40,5 |
| Expansion durant la prise (%) | 0,5 | | 0,1 | | 0,6±0,1 |

La comparaison des résultats montre que la nature de la phase liquide n'a pas d'influence sur la résistance à la compression. Mais la nature de la phase liquide et le rapport L/P agissent sur le temps de prise final et sur le temps d'injection total. Les tampons NaP ou NH4P seront choisis lorsqu'une prise rapide est souhaitée. Un tampon NaGP sera choisi lorsqu'une facilité d'injection est souhaitée.

### Exemple 2

### Préparation de microbilles de PLAGA

On a utilisé un polymère PLA_{37,5}GA₂₅ intrinsèquement amorphe théoriquement constitué par 37,5% d'unités L-lactiques et 25% d'unités glycoliques. Ledit polymère a une température de transition vitreuse de 49°C, un module de conservation E' ≈ 3,5 Gpa, un module de perte E'' ≈ 120 Mpa, un angle de perte δ ≈ 2,0° (à T = 37°C et F = 500 Hz). Les spectres RMN ¹H donnent une composition globale PLA_{36,8}GA_{26,4}. La chromatographie de perméation sur gel donne un seul pic correspondant à une masse molaire **M̅p** de 111 100 avec une distribution **M̅p/M̅n**= 1,9.

4 g dudit polymère ont été mis en solution dans 50 ml de dichlorométhane, et la solution ainsi obtenue a été ajoutée à 1 1 d'une solution aqueuse contenant 5 g d'alcool polyvinylique (PVA) sous une agitation mécanique de 900 rpm. L'agitation a été maintenue pendant 4 h à température ambiante, puis les sphères formées ont été récupérées par filtration sur verre fritté (porosité 3), lavées à l'eau distillée, puis séchées sur buchner. Après une nuit au dessicateur, les sphères ont été tamisées et séparées selon leur taille. Les sphères ayant un diamètre de 125-250 µm (désignées ci-après par microsphères S2) ont été récupérées pour une utilisation ultérieure.

### Exemple 3

### Composition de ciment avec microbilles de polymère pour différentes proportions de polymère

On a reproduit le mode opératoire de l'exemple 1 correspondant aux échantillons 1A et 1C, mais en remplaçant une partie de la phase solide minérale "6 DCPD, 2,5 CaO, 1,5 SrCO₃" par un polymère sous forme de microsphères S2 de l'exemple 2. Dans chaque cas, on a effectué trois essais en faisant varier la quantité de polymère. On a constaté qu'un rapport en masse polymère/phase minérale de 40% est suffisant pour que les microsphères soient en contact, c'est-à-dire pour obtenir des macroporosités interconnectées après dégradation du polymère.

Les microsphères ont été incorporées à la poudre de phosphate, puis le mélange a été incorporé à la phase liquide. La résistance à la compression a été déterminée pour les compositions obtenues respectivement avec NaP et avec NaGP et 40% de microsphères de polymère S2, et comparée à celle de la composition équivalente sans microsphères. Les résultats sont donnés dans le tableau 2 ci-dessous. Dans tous les cas, les compressions ont été déterminées après 24 h à 37°C et 100% d'humidité relative.

**Tableau 2**

| **Echantillon** | **1A** | **3A** | **1C** | **3C** |
|---|---|---|---|---|
| Phase liquide | NaP | NaP | NaGP | NaGP |
| L/P | 0,5 | 0,5 | 0,5 | 0,5 |
| Polymère/phase minérale (% en poids) | 0 | 40 | 0 | 40 |
| Résistance à la compression (MPa) | 21,0±2,8 | 19,6±2 | 19,5±2,3 | 19,2±2 |

Il apparaît ainsi que l'addition de microbilles de polymère ne modifie pratiquement pas la résistance à la compression.

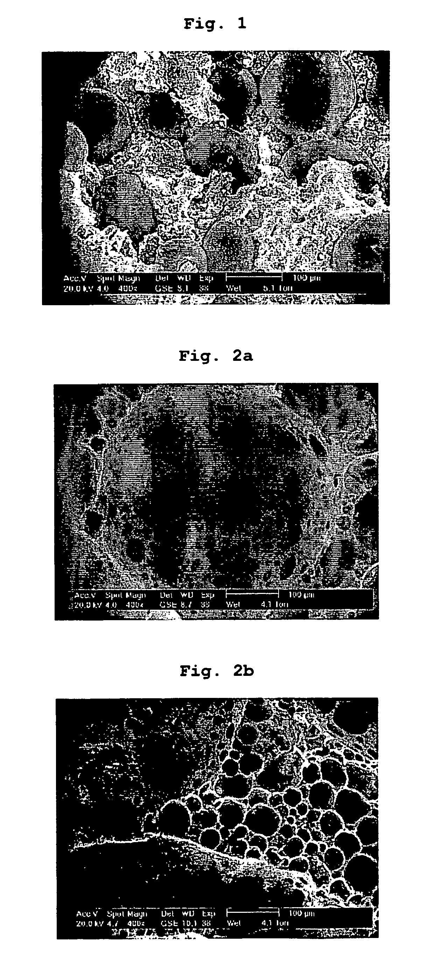

Les figures 1, 2a et 2b représentent des images obtenues par l'observation en microscopie électronique à balayage environnemental (MEBE).

La fig. 1 représente la surface de l'échantillon 3A 1 jour après le mélange de la phase liquide et de la phase solide. Les microsphères de polymère ne sont pas encore dégradées et sont parfaitement visibles, noyées dans la matrice minérale.

Les figures 2a et 2b représentent le coeur de l'échantillon 3A après 33 jours et avant la résorption complète des microsphères. La figure 2a montre une microsphère après rupture de l'échantillon ; les nombreuses vacuoles présentes sur la microsphère indiquent la dégradation hydrolytique avancée du polymère après 33 jours, qui se produit même au sein des échantillons de ciment composite et non seulement en surface.

La figure 2b montre une partie de 3 microsphères (coin supérieur gauche, coin inférieur gauche et coin supérieure droit) entourant une zone minérale alvéolaire ayant une porosité moyenne (diamètre de pore de 5 à 100 µm) importante ; cette porosité moyenne se voit également autour de la microsphère de la figure 2a.

Il apparaît ainsi que, tant à la surface qu'au coeur de l'échantillon de ciment composite 3A, la répartition des microsphères est homogène et les microsphères se touchent, ce qui donnera des macropores interconnectés après résorption du polymère.

### Exemple 4

### Composition de ciment avec microbilles de polymère pour différents rapports L/P

On a reproduit le mode opératoire de l'exemple 1 pour chacun des échantillons 1A à 1D, mais en remplaçant une partie de la phase solide minérale pour un polymère sous forme de microsphères S2, pour avoir un rapport en poids polymère/phase minérale de 40%. Les microsphères ont été incorporées de la même manière que dans l'exemple 3. On a comparé diverses propriétés des quatre échantillons obtenus et le résultat est donné dans le tableau 3 ci dessous. Les compressions ont été déterminées après 24 h à 37°C et 100% d'humidité relative.

**Tableau 3**

| **Echantillon** | **4A = 3A** | **1A** | **4B** | **1B** | **4C = 3C** | **4D** |
|---|---|---|---|---|---|---|
| Phase liquide | NaP | NaP | NaP | NaP | NaGP | NaGP |
| L/P | 0,5 | 0,5 | 0,6 | 0,6 | 0,5 | 0,6 |
| Polymère/phase minérale (% en poids) | 40 | 0 | 40 | 0 | 40 | 40 |
| Résistance à la compression (MPa) | 19.6±2 | 21±2.8 | | 17.6±1.8 | 19.2±2 | 12,8±2,5 |
| Temps d'injection total t_{100%} (min) | 0 | 6 | 5 | 12 | 5 | 7 |

L'on a constaté que l'addition de microbilles diminue fortement le temps de prise et le temps d'injection à 100%. Pour l'échantillon 4A (tampon NaP), le T_{100%} est quasi nul alors qu'il est de 6 min pour le ciment équivalent sans microbilles (Echantillon 1A). De même, pour l'échantillon 1B sans microbilles, le T_{100%} qui est de 12 min passe à 5 min pour une teneur en microbilles de 40%. Le remplacement du tampon NaP par le tampon NaGP améliore l'injectabilité du ciment composite.

### Exemple 5

### Dégradation d'un ciment composite de l'exemple 3

Des échantillons d'un ciment sans microbilles 1A (désigné ci-après par "ciment" et d'un ciment avec microbilles 3A (désigné ci-après par composite) ont été immergés, 1 heure après leur préparation, dans 10 ml d'eau ou d'un tampon phosphate de sodium pH 7, 0,05 M.

Dans une première série, les échantillons sont restés pendant un mois dans la même solution dont le pH a été mesuré journellement. Chaque semaine, leur surface a été examinée par MEBE et la présence éventuelle d'oligomères provenant de la dégradation du PLAGA a été recherchée par électrophorèse capillaire de zone.

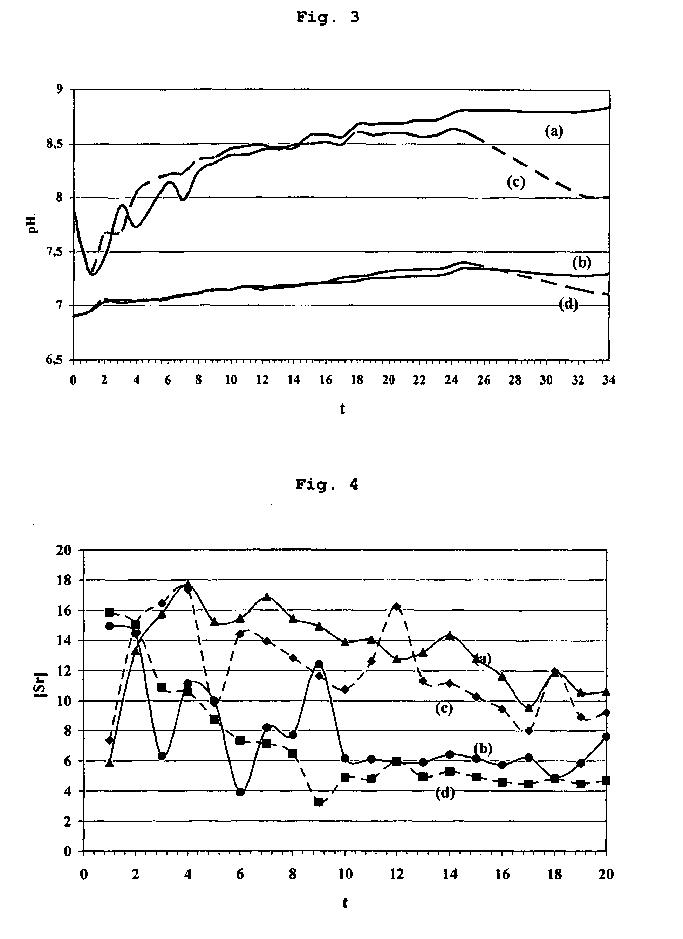

Pour les échantillons de ciment et pour les échantillons de composite, le pH de l'eau croît lentement avec le temps de contact pour se stabiliser après 4 semaines à une valeur des 8,8 (pour le ciment) ou décroître (pour le composite). Le même comportement est observé en tampon phosphate, mais avec une plus faible variation. L'évolution du pH est représentée sur la figure 3. Le temps t (en jours) est indiqué en abscisse, le pH est indiqué en ordonnée. La correspondance entre les échantillons et les solutions de trempage d'une part et les courbes d'autre part, est comme suit :

| | |
|---|---|
| Courbe a | ciment dans l'eau |
| Courbe b | ciment dans le tampon phosphate |
| Courbe c | composite dans l'eau |
| Courbe d | composite dans le tampon phosphate. |

La chute du pH après le 24^{ème} jour visible sur les courbes c et d indique la dégradation accélérée des microsphères de polymère dans l'eau comme dans le tampon phosphate de sodium, ce qui acidifie le milieu avec pour résultat d'accélérer la dégradation de la partie minérale.

La libération d'ions calcium à partir du ciment et du composite est très faible aussi bien dans l'eau qu'en tampon phosphate (en limite de détection). L'analyse d'une solution inchangée pendant un mois donne des concentrations de 11 ppm dans le tampon et 18 ppm dans l'eau, soit une moyenne par jour de 0,4 à 0,6 ppm.

Dans une deuxième série, la solution a été changée chaque jour et la concentration en ions strontium, calcium et phosphate a été mesurée.

La libération d'ions phosphate dans l'eau à partir du ciment et du composite n'est significative que les premiers jours. Elle est très faible les jours suivants (environ 0,5 ppm/jour). Les ions phosphate libérés en grande quantité les premier jours proviennent du tampon utilisé comme phase liquide comme cela a été montré pour le ciment uniquement composé de DCPD et de CaO (S. Munier, et al, Bioceramics 16, Key Engineering Materials, Vol 254-259, 2004, p. 615-618, Trans Tech Publications, Suisse).

La libération d'ions strontium à partir du ciment et du composite est beaucoup plus importante que celle des ions calcium, aussi bien dans l'eau qu'en tampon phosphate. Elle est continue et se stabilise autour de 5 ppm en tampon phosphate, et 10 ppm dans l'eau. Cette libération n'est pas statistiquement différente à partir du composite ou du ciment. Le facteur de probabilité P, déterminé selon la méthode ANOVA 1 disponible sur le logiciel Excel@ de la société Microsoft, est égal à 0,16, bien que la présence de microsphères de PLAGA diminue la quantité de strontium libérable. Elle est cependant statistiquement plus faible en tampon phosphate que dans l'eau (P = 0,008). Les courbes de la figure 4 représentent l'évolution de la concentration en ions Sr²⁺ dans un bain d'immersion changé chaque jour. Le temps t (en jours), est donné en abscisse, la concentration en ions Sr²⁺ [Sr] en ppm/jour, est donnée en ordonnée. Le bain d'immersion est soit de l'eau pure apyrogène, soit un tampon phosphate constitué par une solution à 0,05 mol/l d'un mélange de NaH₂PO₄ et de Na₂HPO₄, 12H₂O de pH 7.

La correspondance entre les échantillons et les solutions de trempage d'une part et les courbes d'autre part, est comme suit :

| | |
|---|---|
| Courbe a) matérialisée par ▲ | ciment dans l'eau |
| Courbe b) matérialisée par ● | ciment dans le tampon phosphate |
| Courbe c) matérialisée par ◆ | composite dans l'eau |
| Courbe d) matérialisée par ■ | composite dans le tampon phosphate |

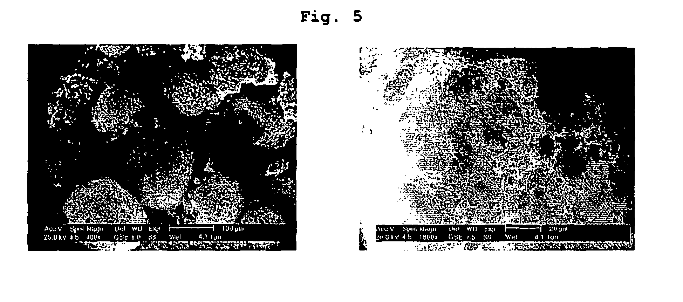

La présence des oligomères (G, L, G₂, L/G, G/L, L₂), qui indique une dégradation du polymère PLAGA au stade monomère ou dimère soluble, n'est réellement mise en évidence qu'à partir du vingt quatrième jour, date qui correspond au début de décroissance du pH des solutions en présence de composite, bien que l'observation en MEBE montre une évolution très nette de la surface des microsphères dès le 11^{ème} jour (coupure des longues chaînes en chaînes plus courtes). La figure 5 représente l'aspect du ciment composite montrant l'état des sphères après une dégradation de 11 jours (photo de gauche) et 18 jours (photo de droite). La comparaison avec la figure 1 montre bien l'évolution des microsphères qui sont très nettes après 1 jour, qui présentent des irrégularités après 11 jours et un aspect d'éponge après 18 jours. La présence d'oligomères et de monomères et la chute du pH après 24 jours indiquent une dégradation accélérée du polymère à partir du 24^{ème} jour : le polymère gonfle, ce qui a entraîné l'éclatement des plots après 45 jours.

## Revendications

1. Composition pour ciment injectable, **caractérisée en ce qu'**elle comprend une phase solide minérale et une phase liquide séparée, et éventuellement des microparticules d'un polymère biocompatible et biodégradable ;
- la phase solide minérale étant constituée par un mélange de poudres ayant la composition molaire (CP)₆ (CaO)_{y} (SrCO₃)_{z}, dans laquelle CP représente l'hydrogénophosphate de calcium dihydraté CaHPO₄, 2H₂O (DCPD), l'hydrogénophosphate de calcium anhydre CaHPO₄ (DCPA), l'hydrogénophosphate mixte de calcium et de strontium anhydre (Ca₁₋ₓSrₓ) HPO₄, un mélange équimolaire de bis-dihydrogénophosphate de calcium monohydraté Ca(H₂PO₄)₂, H₂O (MCPM) et d'oxyde de calcium CaO, ou un mélange de deux ou trois de ces composés, et y+z = 4±1 ;
- la phase liquide étant constituée par de l'eau pure apyrogène ou par une solution aqueuse saline ayant un pH de 4 à 9 ;
le rapport L/P [volume de phase liquide /masse de phase solide minérale] étant au moins égal à 0,4 ml/g.

2. Composition selon la revendication 1, **caractérisé en ce que** CP représente CaHPO₄, 2H₂O.

3. Composition selon la revendication 1, **caractérisé en ce que** CP est un mélange comprenant CaHPO₄, 2H₂O.

4. Composition pour ciment injectable selon la revendication 1, **caractérisée en ce que** la phase liquide est une solution contenant de 0,4 à 1 mol/l d'un mélange de NaH₂PO₄ et de Na₂HPO₄, 12H₂O.

5. Composition pour ciment selon la revendication 1, **caractérisée en ce que** la phase liquide est une solution contenant de 0,4 à 1 mol/l d'un mélange de NaH₂PO₄ et de glycérophosphate de sodium hexahydraté Na₂PO₄C₃H₇O₂,6H₂O.

6. Composition pour ciment injectable selon la revendication 1, **caractérisée en ce que** la phase liquide est une solution aqueuse contenant de 0,4 à 1,5 mol/l d'un mélange de NH₄H₂PO₄ et de (NH₄)₂HPO₄.

7. Composition pour ciment injectable selon la revendication 1, **caractérisée en ce que** la phase solide minérale contient des microparticules d'un polymère biocompatible et biodégradable.

8. Composition pour ciment injectable selon la revendication 1, **caractérisée en ce qu'**elle comprend une troisième phase constituée par des microparticules d'un polymère biocompatible et biodégradable.

9. Composition pour ciment injectable selon l'une des revendications 7 et 8, **caractérisée en ce que** le polymère est un polymère PLAGA intrinsèquement amorphe, constitué par des unités L-lactique, des unités D-lactique, et des unités glycoliques, ou un polymère PLA constitué uniquement par des unités L-lactiques, ou un copolymère d'acide lactique, d'acide glycolique et d'acide gluconique.

10. Composition pour ciment injectable selon l'une des revendications 7 et 8, **caractérisée en ce que** le rapport massique du polymère à la phase solide minérale est compris entre 20 et 60 %.

11. Composition pour ciment injectable selon la revendication 1, **caractérisée en ce qu'**au moins une partie du carbonate de strontium est du carbonate de strontium 89 radioactif.

12. Composition pour ciment injectable selon la revendication 1, ne contenant pas de microparticules de polymère, **caractérisé en ce que** le rapport L/P est de 0,4 ml/g à 0,7 ml/g.

13. Composition pour ciment injectable selon la revendication 1, contenant des microparticules de polymère, **caractérisé en ce que** le rapport L/P est supérieur ou égal à 0,6 ml/g.

14. Procédé de préparation d'un ciment injectable à partir d'une composition selon la revendication 1, **caractérisé en ce qu'**il consiste à mettre en contact la phase solide et la phase liquide de la composition pour ciment injectable, au moment de l'utilisation du ciment injectable, dans les conditions d'asepsie requises pour l'injection de substituts osseux.

15. Procédé selon la revendication 14, **caractérisé en ce que** l'on utilise une composition selon la revendication 7 dans laquelle la phase solide contient des microparticules de polymère.

16. Procédé selon la revendication 14, **caractérisé en ce que** l'on utilise une composition selon la revendication 8, comprenant une troisième phase constituée par des microparticules de polymère, ladite 3^{ème} phase étant ajoutée après mise en contact de la phase solide minérale et de la phase liquide.

17. Ciment injectable obtenu par un procédé selon la revendication 14, **caractérisé en ce qu'**il est constitué par (CP), (CaO) (SrCO₃), [CP représentant l'hydrogénophosphate de calcium dihydraté CaHPO₄, 2H₂O (DCPD), l'hydrogénophosphate de calcium anhydre CaHPO₄ (DCPA), l'hydrogénophosphate mixte de calcium et de strontium anhydre (Ca₁₋ₓSrₓ)HPO₄], un mélange équimolaire de bis-dihydrogénophosphate de calcium monohydraté Ca(H₂PO₄)₂, H₂O (MCPM) et d'oxyde de calcium CaO, ou un mélange de ces constituants), de l'eau et les sels éventuellement contenus dans la phase liquide, les quantités respectives étant telles que :
- le rapport molaire (CP) / (CaO) / (SrCO₃) est de 6/y/z avec y+z = 4±1.
- le rapport liquide (volume) / solide (masse) est de 0,4 à 0,7 ml/g, plus particulièrement de 0,5 à 0,6 ml/g.

18. Ciment injectable selon la revendication 17, **caractérisé en ce que** CP représente CaHPO₄, 2H₂O.

19. Ciment injectable obtenu par le procédé selon la revendication 15 ou 16, **caractérisé en ce qu'**il est constitué par (CP), (CaO) (SrCO₃), [CP représentant l'hydrogénophosphate de calcium dihydraté CaHPO₄, 2H₂O (DCPD), l'hydrogénophosphate de calcium anhydre CaHPO₄ (DCPA), l'hydrogénophosphate mixte de calcium et de strontium anhydre (Ca₁₋ₓSrₓ)HPO₄], un mélange équimolaire de bis-dihydrogénophosphate de calcium monohydraté Ca(H₂PO₄₎₂, H₂O (MCPM) et d'oxyde de calcium CaO, ou un mélange de ces constituants), des microparticules de polymère, de l'eau et les sels éventuellement contenus dans la phase liquide, les quantités respectives étant telles que :
- le rapport molaire (CP) / (CaO) / (SrCO₃) est de 6/y/z avec y+z = 4±1,
- le rapport L/P initial est supérieur ou égal à 0,6 ml/g ;
- le rapport massique polymère/phase minérale solide est entre 20 et 60%.

20. Ciment injectable selon la revendication 17 ou 19, pour le traitement de l'ostéoporose ou pour la vertébroplastie.

21. Ciment injectable selon la revendication 17 ou 19, pour le comblement osseux suite à une fracture ou une exérèse.

## Claims

1. A composition for injectable cement, **characterized in that** it comprises a separate mineral solid phase and a separate liquid phase, and optionally microparticles of a biocompatible and biodegradable polymer;
- the mineral solid phase being composed of a mixture of powders having the molar composition (CP)₆(CaO)y(SrCO₃)_{z}, in which CP represents calcium hydrogenphosphate dihydrate CaHPO₄·2H₂O (DCPD), anhydrous calcium hydrogenphosphate CaHPO₄ (DCPA), anhydrous mixed calcium strontium hydrogenphosphate (Ca₁₋ₓSrₓ)HPO₄, an equimolar mixture of calcium bis(dihydrogenphosphate) monohydrate Ca(H₂PO₄)₂·H₂O (MCPM) and of calcium oxide CaO, or a mixture of two or three of these compounds, and y + z = 4 ± 1;
- the liquid phase being composed of pure pyrogen-free water or of an aqueous saline solution having a pH of 4 to 9;
the L/P [volume of liquid phase/weight of mineral solid phase] ratio being at least equal to 0.4 ml/g.

2. The composition as claimed in claim 1, **characterized in that** CP represents CaHPO₄·2H₂O.

3. The composition as claimed in claim 1, **characterized in that** CP is a mixture comprising CaHPO₄·2H₂O.

4. The composition for injectable cement as claimed in claim 1, **characterized in that** the liquid phase is a solution comprising from 0.4 to 1 mol/l of a mixture of NaH₂PO₄ and of Na₂HPO₄·12H₂O.

5. The composition for cement as claimed in claim 1, **characterized in that** the liquid phase is a solution comprising from 0.4 to 1 mol/l of a mixture of NaH₂PO₄ and of sodium glycerophosphate hexahydrate Na₂PO₄C₃H₇O₂·6H₂O.

6. The composition for injectable cement as claimed in claim 1, **characterized in that** the liquid phase is an aqueous solution comprising from 0.4 to 1.5 mol/l of a mixture of NH₄H₂PO₄ and of (NH₄)₂HPO₄.

7. The composition for injectable cement as claimed in claim 1, **characterized in that** the mineral solid phase comprises microparticles of a biocompatible and biodegradable polymer.

8. The composition for injectable cement as claimed in claim 1, **characterized in that** it comprises a third phase composed of microparticles of a biocompatible and biodegradable polymer.

9. The composition for injectable cement as claimed in either of claims 7 and 8, **characterized in that** the polymer is an intrinsically amorphous PLAGA polymer composed of L-lactic units, D-lactic units and glycolic units, or a PLA polymer composed solely of L-lactic units, or a copolymer of lactic acid, of glycolic acid and of gluconic acid.

10. The composition for injectable cement as claimed in either of claims 7 and 8, **characterized in that** the ratio by weight of the polymer to the mineral solid phase is between 20 and 60%.

11. The composition for injectable cement as claimed in claim 1, **characterized in that** at least a portion of the strontium carbonate is radioactive strontium-89 carbonate.

12. The composition for injectable cement as claimed in claim 1, not comprising polymer microparticles, **characterized in that** the L/P ratio is from 0.4 ml/g to 0.7 ml/g.

13. The composition for injectable cement as claimed in claim 1 comprising polymer microparticles, **characterized in that** the L/P ratio is greater than or equal to 0.6 ml/g.

14. A process for the preparation of an injectable cement from a composition as claimed in claim 1, **characterized in that** it consists in bringing the solid phase and the liquid phase of the composition for injectable cement into contact, at the time of the use of the injectable cement, under the aseptic conditions required for the injection of bone replacements.

15. The process as claimed in claim 14, **characterized in that** use is made of a composition as claimed in claim 7 in which the solid phase comprises polymer microparticles.

16. The process as claimed in claim 14, **characterized in that** use is made of a composition as claimed in claim 8 comprising a third phase composed of polymer microparticles, said 3rd phase being added after the mineral solid phase and the liquid phase have been brought into contact.

17. An injectable cement obtained by a process as claimed in claim 14, **characterized in that** it is composed of (CP)(CaO)(SrCO₃) [CP representing calcium hydrogenphosphate dihydrate CaHPO₄·2H₂O (DCPD), anhydrous calcium hydrogenphosphate CaHPO₄ (DCPA), anhydrous mixed calcium strontium hydrogenphosphate (Ca₁₋ₓSrₓ)HPO₄, an equimolar mixture of calcium bis(dihydrogenphosphate) monohydrate Ca(H₂PO₄)₂·H₂O (MCPM) and of calcium oxide CaO, or a mixture of these constituents], water and the salts possibly present in the liquid phase, the respective amounts being such that:
- the (CP)/(CaO)/(SrCO₃) molar ratio is 6/y/z with y + z = 4 ± 1,
- the liquid (volume)/solid (weight) ratio is from 0.4 to 0.7 ml/g, more particularly from 0.5 to 0.6 ml/g.

18. The injectable cement as claimed in claim 17, **characterized in that** CP represents CaHPO₄·2H₂O.

19. An injectable cement obtained by the process as claimed in claim 15 or 16, **characterized in that** it is composed of (CP)(CaO)(SrCO₃) [CP representing calcium hydrogenphosphate dihydrate CaHPO₄·2H₂O (DCPD), anhydrous calcium hydrogenphosphate CaHPO₄ (DCPA), anhydrous mixed calcium strontium hydrogenphosphate (Ca₁₋ₓSrₓ)HPO₄, an equimolar mixture of calcium bis(dihydrogenphosphate) monohydrate Ca(H₂PO₄)₂·H₂O (MCPM) and of calcium oxide CaO, or a mixture of these constituents], polymer microparticles, water and the salts possibly present in the liquid phase, the respective amounts being such that:
- the (CP)/(CaO)/(SrCO₃) molar ratio is 6/y/z with y + z = 4 ± 1,
- the initial L/P ratio is greater than or equal to 0.6 ml/g;
- the polymer/mineral solid phase ratio by weight is between 20 and 60%.

20. The injectable cement as claimed in claim 17 or 19, for the treatment of osteoporosis or in spondylotherapy.

21. The injectable cement as claimed in claim 17 or 19 for filling in bone subsequent to a fracture or an excision.

## Patentansprüche

1. Zusammensetzung für injizierbaren Zement, **dadurch gekennzeichnet, dass** sie eine mineralische feste Phase und eine getrennte flüssige Phase umfasst und eventuell Mikropartikel eines biologisch verträglichen und biologisch abbaubaren Polymers,
- wobei die mineralische feste Phase von einem Pulvergemisch mit der molaren Zusammensetzung (CP)₆ (CaO)_{y} (SrCO₃)_{z} gebildet wird, wobei CP Calciumhydrogenphosphatdihydrat CaHPO₄, 2H₂O (DCPD), wasserfreies Calciumhydrogenphosphat CaHPO₄ (DCPA), wasserfreies Calcium- und Strontiumhydrogenphosphatgemisch (Ca₁₋ₓSrₓ) HPO₄, ein äquimolares Gemisch aus Calcium-bis-dihydrogenphosphat Monohydrat Ca (H₂PO₄)₂, H₂O (MCPM) und Calciumoxid CaO, oder ein Gemisch von zwei oder drei dieser Verbindungen, und y+z = 4±1 darstellt,
- die flüssige Phase aus pyrogenfreiem reinem Wasser oder einer wässrigen Salzlösung mit einem pH von 4 bis 9 besteht,
wobei das Verhältnis L/P [Volumen der flüssigen Phase / Masse der mineralischen festen Phase] mindestens gleich 0,4 ml/g ist.

2. Zusammensetzung nach Anspruch 1, **dadurch gekennzeichnet, dass** CP CaHPO₄, 2H₂O darstellt.

3. Zusammensetzung nach Anspruch 1, **dadurch gekennzeichnet, dass** CP ein Gemisch ist, das CaHPO₄, 2H₂O umfasst.

4. Zusammensetzung für injizierbaren Zement nach Anspruch 1, **dadurch gekennzeichnet, dass** die flüssige Phase eine Lösung ist, die 0,4 bis 1 mol/l eines Gemischs aus NaH₂PO₄ und Na₂HPO₄, 12H₂O enthält.

5. Zusammensetzung für Zement nach Anspruch 1, **dadurch gekennzeichnet, dass** die flüssige Phase eine Lösung ist, die 0,4 bis 1 mol/l eines Gemischs aus NaH₂PO₄ und Natriumglycerophosphat-Hexahyrat Na₂PO₄C₃H₇O₂, 6H₂O enthält.

6. Zusammensetzung für injizierbaren Zement nach Anspruch 1, **dadurch gekennzeichnet, dass** die flüssige Phase eine wässrige Lösung ist, die 0,4 bis 1,5 mol/l eines Gemischs aus NH₄H₂PO₄ und (NH₄)₂HPO₄ enthält.

7. Zusammensetzung für injizierbaren Zement nach Anspruch 1, **dadurch gekennzeichnet**, das die mineralische feste Phase Mikropartikel eines biologisch verträglichen und biologisch abbaubaren Polymers enthält.

8. Zusammensetzung für injizierbaren Zement nach Anspruch 1, **dadurch gekennzeichnet, dass** sie eine dritte Phase umfasst, die von Mikropartikeln eines biologisch verträglichen und biologisch abbaubaren Polymers gebildet wird.

9. Zusammensetzung für injizierbaren Zement nach einem der Ansprüche 7 und 8, **dadurch gekennzeichnet, dass** das Polymer ein immanent amorphes PLAGA-Polymer ist, das von L-Milchsäureeinheiten, D-Milchsäureeinheiten und Glycoleinheiten gebildet wird, oder ein PLA-Polymer, das ausschließlich von L-Milchsäureeinheiten gebildet wird, oder ein Copolymer aus Milchsäure, Glycolsäure und Gluconsäure.

10. Zusammensetzung für injizierbaren Zement nach einem der Ansprüche 7 und 8, **dadurch gekennzeichnet, dass** das Massenverhältnis des Polymers in der mineralischen festen Phase zwischen 20 und 60 % inklusive ist.

11. Zusammensetzung für injizierbaren Zement nach Anspruch 1, **dadurch gekennzeichnet, dass** mindestens ein Teil des Strontiumscarbonats radioaktives Strontium-89-Carbonat ist.

12. Zusammensetzung für injizierbaren Zement nach Anspruch 1, die keine Polymer-Mikropartikel enthält, **dadurch gekennzeichnet, dass** das Verhältnis L/P 0,4 ml/g bis 0,7 ml/g ist.

13. Zusammensetzung für injizierbaren Zement nach Anspruch 1, die Polymer-Mikropartikel enthält, **dadurch gekennzeichnet, dass** das Verhältnis L/P größer oder gleich 0,6 ml/g ist.

14. Verfahren zur Zubereitung eines injizierbaren Zements aus einer Zusammensetzung nach Anspruch 1, **dadurch gekennzeichnet, dass** es darin besteht, die feste Phase und die flüssige Phase der Zusammensetzung für injizierbaren Zement im Moment der Verwendung des injizierbaren Zements unter den für die Injektion von Knochenersatz erforderlichen aseptischen Bedingungen in Kontakt zu bringen.

15. Verfahren nach Anspruch 14, **dadurch gekennzeichnet, dass** eine Zusammensetzung nach Anspruch 7 verwendet wird, wobei die feste Phase Polymer-Mikropartikel enthält.

16. Verfahren nach Anspruch 14, **dadurch gekennzeichnet, dass** eine Zusammensetzung nach Anspruch 8 verwendet wird, die eine dritte Phase umfasst, die von Polymer-Mikropartikeln gebildet wird, wobei die dritte Phase hinzugefügt wird, nachdem die mineralische feste Phase und die flüssige Phase in Kontakt gebracht wurden.

17. Injizierbarer Zement, der durch ein Verfahren nach Anspruch 14 hergestellt wird, **dadurch gekennzeichnet, dass** er aus (CP), (CaO) (SrCO₃), [wobei CP Calciumhydrogenphosphatdihydrat CaHPO₄, 2H₂O (DCPD), wasserfreies Calciumhydrogenphosphat CaHPO₄ (DCPA), wasserfreies Calcium- und Strontiumhydrogenphosphatgemisch (Ca₁₋ₓSrₓ) HPO₄ darstellt], einem äquimolaren Gemisch aus Calcium-bis-dihydrogenphosphat Monohydrat Ca (H₂PO₄)₂, H₂O (MCPM) und Calciumoxid CaO oder einem Gemisch dieser Bestandteile), Wasser und eventuell in der flüssigen Phase enthaltenen Salzen besteht, wobei die entsprechenden Mengen derart sind, dass:
- das molare Verhältnis (CP) / (CaO) / (SrC03) 6/y/z mit y+z = 4+1 beträgt,
- das Verhältnis Flüssigkeit (Volumen) / Feststoff (Masse) 0,4 bis 0,7 ml/g, insbesondere 0,5 bis 0,6 ml/g beträgt.

18. Injizierbarer Zement nach Anspruch 17, **dadurch gekennzeichnet, dass** CP CaHPO₄, 2H₂O darstellt.

19. Injizierbarer Zement, der durch das Verfahren nach Anspruch 15 oder 16 hergestellt wird, **dadurch gekennzeichnet, dass** er aus (CP), (CaO) (SrCO₃) [wobei CP Calciumhydrogenphosphatdihydrat CaHPO₄, 2H₂O (DCPD), wasserfreies Calciumhydrogenphosphat CaHPO₄ (DCPA), wasserfreies Calcium- und Strontiumhydrogenphosphatgemisch (Ca₁₋ₓSrₓ) HPO₄ darstellt], einem äquimolaren Gemisch aus Calcium-bis-dihydrogenphosphat Monohydrat Ca (H₂PO₄)₂, H₂O (MCPM) und Calciumoxid CaO, oder einem Gemisch dieser Bestandteile), Polymer-Mikropartikeln, Wasser und eventuell in der flüssigen Phase enthaltenen Salzen besteht, wobei die entsprechenden Mengen derart sind, dass:
- das molare Verhältnis (CP) / (CaO) / (SrC03) 6/y/z mit y+z = 4+1 beträgt,
- das ursprüngliche Verhältnis L/P größer oder gleich 0,6 ml/g ist,
- das Massenverhältnis Polymer/mineralische feste Phase zwischen 20 und 60 % ist.

20. Injizierbarer Zement nach Anspruch 17 oder 19 zur Behandlung der Osteoporose oder für die Vertebroplastik.

21. Injizierbarer Zement nach Anspruch 17 oder 19 für den Knochenaufbau nach einer Fraktur oder einer Exzision.
